# FASCICULE DE BREVET EUROPEEN

(11) **EP 0 765 877 B1**
(45) Date de publication et mention de la délivrance du brevet: **17.12.2003**
(21) Numéro de dépôt: 96402044.0
(22) Date de dépôt: 26.09.1996
(51) Int. Cl.: C07D 307/92, C07D 405/12, A61K 31/34

(54) **Composés aminés de 5,6,7,8-tétrahydronaphto(2,3-b)furane et d'indano(5,6-b)furane, leurs procédés de préparation et compositions pharmaceutiques qui les contiennent**
5,6,7,8-Tetrahydronaphto(2,3-b)furan- und indano(5,6-b)furan-amino-Verbindungen, Verfahren zu deren Herstellung und sie enthaltende pharmazeutische Zusammensetzungen
5,6,7,8-Tetrahyronaphto(2,3-b)furan- and indano(5,6-b)furan-amino-compounds, processes for their preparation and pharmaceutical compositions containing them

(30) Priorité: 29.09.1995 FR 9511446
(43) Date de publication de la demande: 02.04.1997
(73) Titulaire: LES LABORATOIRES SERVIER, 92200 Neuilly sur Seine (FR)
(72) Inventeur: Peglion, Jean-Louis, 78110 Le Vesinet (FR); Vian, Joel, 92370 Chaville (FR); Dessinges, Aimée, 94320 Thiais (FR); Millan, Mark, 75017 Paris (FR); Audinot, Valérie, 78300 Poissy (FR)

(56) Documents cités:
- EP-A- 0 286 515
- EP-A- 0 286 516
- US-A- 4 470 990

## Description

La présente invention a pour objet de nouveaux composés aminés de 5,6,7,8-tétrahydronaphto[2,3-b]furane et d'indano[5,6-b]furane, leurs procédés de préparation et les compositions pharmaceutiques qui les contiennent.

Elle concerne plus spécialement les composés aminés de 5,6,7,8-tétrahydronaphto[2,3-b] furane et d'indano[5,6-b]furane de formule I : 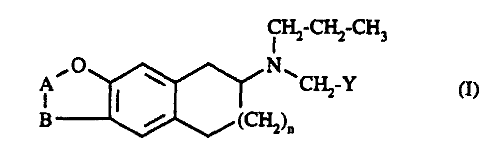 dans laquelle :
- **A-B**: représente CH=CH ou CH₂-CH₂ ;
- **n**: représente zéro ou un ; et
- **Y**: représente :
- un radical ω-(cycloalkyl)-alkyle, ω-(méthyl cycloalkyl)-alkyle ou ω,ω-(di-cycloalkyl)-alkyle dans chacun desquels chaque groupe cycloalkyl renferme de 3 à 7 atomes de carbone et la partie alkyle renferme de 1 à 4 atomes de carbone en chaîne droite ou ramifiée ; ou
- un radical de formule : 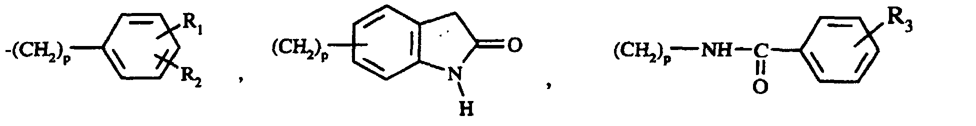 et 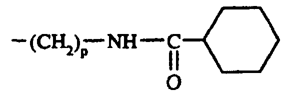
dans lesquelles :
p est un nombre entier de 1 à 4 inclus,
R₁ représente un atome d'hydrogène ou d'halogène, ou un radical hydroxy ou méthoxy,
R₂ représente un atome d'halogène, un radical hydroxy, (C₁-C₅)alkoxy ou phényle ou un groupement de formule : -NH-CO-CH₃, -NH-CO-CF₃ et -NH-SO₂-CH₃, et
R₃ représente un atome d'halogène ou un radical (C₁-C₅)alkoxy, trifluorométhyle, cyano, phényle, p-aminophényle ou p-acétylphényle ;
sous forme racémique ou d'isomères optiques,
et leurs sels d'addition avec un acide pharmaceutiquement acceptable.

L'état antérieur de la technique est illustré notamment par les brevets EP 0 286 515 et 0 286 516 qui concernent, entre autres, des dérivés aminés du 5,6,7,8-tétrahydronaphto [2,3-b] furane se comportant comme des substances dopaminergiques et présentant une activité antidépressive, anti-agressive et psychostimulante.

Des recherches effectuées dans les services de la demanderesse ont montré qu'en modifiant les substituants de la fonction amine, on parvenait à renforcer les propriétés dopaminergiques de ces produits tout en les rendant plus spécifiques et sélectives, ce qui permet aux dits produits de présenter moins d'effets secondaires.

Actuellement, les substances utilisées en thérapeutique pour le traitement des affections dans lesquelles est impliqué le système dopaminergique, ne présentent pas de sélectivité et se lient toutes très fortement au récepteur D₂, qu'il s'agisse des bloqueurs dopaminergiques (utilisés dans les troubles liés à l'hyperactivité de ce neurotransmetteur tels qu'ils se présentent, par exemple, dans la schizophrénie) ou d'activateurs dopaminergiques (utilisés dans les troubles liés à l'hypoactivité tels qu'ils se présentent dans la maladie de Parkinson par exemple). Toutefois, ces bloqueurs ou activateurs dopaminergiques D₂ présentent de nombreux effets secondaires : dyskinésie tardive, hyperprolactinémie, aménorrhée pour les premiers, effets cardio-vasculaires et moteurs pour les derniers.

La découverte récente d'un nouveau récepteur à la dopamine, dénommé le récepteur D₃, dont la concentration est très importante au niveau du système limbique, mais très faible dans le noyau nigrostrié et dans les cellules lactotrophes, encourage la recherche de nouveaux médicaments agissant au niveau du système dopaminergique, mais ayant pour cible préférentielle le récepteur D₃ et donc exempt des effets secondaires liés typiquement au récepteur D₂ comme mentionné précédemment.

Les modifications de structures des produits de l'état antérieur de la technique précédemment cité ont abouti aux composés objets de la présente invention qui se différencient des produits des brevets EP 0 286 515 et 0 286 516 à la fois par leur structure chimique et par leurs propriétés pharmacologiques et thérapeutiques.

En effet, des études réalisées in vitro (binding sur récepteurs clonés D₂ et D₃ humains) avec les composés de la présente invention montrent que ceux-ci se comportent comme des ligands très affins au niveau des récepteurs dopaminergiques D₃ tout en ne présentant que peu d'affinité pour les récepteurs dopaminergiques D₂, ce qui n'est par le cas des composés objets des brevets EP 0 286 515 et 0 286 516.

Cette sélectivité confère aux composés de la présente invention un intérêt tout particulier pour leur utilisation comme médicament agissant au niveau du système dopaminergique dans la maladie de Parkinson (J. Neur. Transm., 1993, 94, 11-19), les troubles de la mémoire (Nature, 1990, 347, 146-151), l'abus de drogue (Science, 1993, 260, 1814), la dépression et comme antipsychotique.

La présente invention a également pour objet le procédé de préparation des composés de formule I caractérisé en ce que l'on fait réagir l'amine secondaire de formule II : 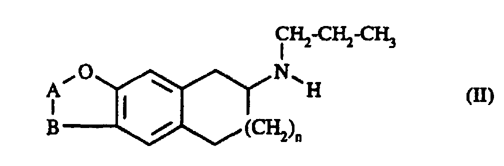 dans laquelle A-B et n ont les significations précédemment définies, avec
- soit un composé halogéné de formule III :

   X-CH₂-Y (III)

   dans laquelle :
   - Y: a la signification précédemment définie et
   - X: représente un atome de chlore, brome ou iode,
   en opérant en milieu alcalin tel que Na₂CO₃/H₂O ou K₂CO₃/CH₃CN
- soit avec un composé de formule IV : 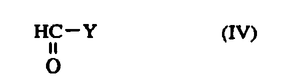 dans laquelle Y a la signification précédemment définie,
   en opérant en milieu réducteur par exemple en présence de triacétoxy borohydrure de sodium dans l'acide acétique ;
- soit avec un composé acylant de formule V : 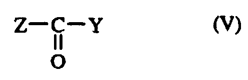 dans laquelle :
   - Y: a la signification précédemment définie et
   - Z: représente un radical hydroxy ou un atome de chlore,
   et l'on réduit le composé obtenu de formule VI : 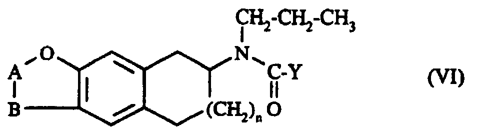
dans laquelle A-B, n et Y ont les significations précédemment définies.

Il est particulièrement avantageux d'effectuer l'acylation du composé de formule II avec le composé de formule V en opérant dans le cas où Z représente un radical hydroxy en milieu dichlorométhane en présence de carbonyl diimidazole et dans le cas ou Z représente un atome de chlore en milieu dichlorométhane en présence de N,N-diisopropyl-éthylamine. La réduction du composé de formule VI s'effectue de façon adéquate en employant comme agent de réduction soit l'hydrure double de lithium et d'aluminium (LiAlH₄) soit le borane-sulfure de diméthyle.

Les matières premières de formule II ont été préparées à partir des amines primaires correspondantes elles-mêmes obtenues selon le procédé décrit dans le brevet US 4 874 878 pour la préparation du dl-7-amino 2,3,5,6,7,8-hexahydronaphto[2,3-b]furane.

Les formes optiquement actives des composés de formule I ont été obtenues soit à partir des formes optiquement actives des matières premières de formule II, soit par dédoublement des formes racémiques des composés de formule I, selon des méthodes connues de la littérature.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par la voie orale, rectale ou parentérale.

La posologie peut varier selon l'âge et le poids du patient, la voie d'administration, la nature de la maladie et les traitements associés et s'échelonne de 0,5 à 25 mg de principe actif, 1 à 3 fois par jour.

Les exemples suivants, donnés à titre non-limitatif, illustrent la présente invention. Les points de fusion ont été déterminés soit à la platine chauffante de Kofler (K), soit à la platine chauffante sous microscope (MK). Les spectres de résonnance magnétique nucléaire du proton (RMN) ont été réalisés à 200 MHz, sauf indication contraire.

### Exemple 1

### (7RS) 7-{N-[2-(3,4-diméthoxyphényl)éthyl]-N-propyl-amino}-5,6,7,8-tétrahydronaphto [2,3-b] 2,3-dihydrofurane, et son dibenzoyltartrate (dl)

### Stade A : (7RS) N-propyl N-(5,6,7,8-tétrahydronaphto [2,3-b] 2,3-dihydrofuran-7-yl) (3,4-diméthoxyphényl)acétamide.

A 4,3 g d'acide 3,4-diméthoxyphényl acétique en solution dans 50 ml de dichlorométhane, on ajoute par portion 3,6 g de carbonyldiimidazole. Après 2 heures d'agitation à température ambiante, on coule goutte à goutte une solution de 5 g de 7-N-propylamino 5,6,7,8-tétrahydronaphto [2,3-b] 2,3-dihydrofurane dans 50 ml de dichlorométhane. Après 18 heures d'agitation à température ambiante, le mélange réactionel est lavé successivement par une solution de soude N, une solution d'acide chlorhydrique N, et une solution saturée de chlorure de sodium. Après décantation et évaporation du solvant à l'évaporateur rotatif, on obtient 4,5 g d'une huile correspondant au composé attendu. Rendement : 55 %.

### Stade B : Composé titre

Une solution de 4,5 g du composé obtenu au stade A dans 80 ml de tétrahydrofurane est ajoutée goutte à goutte à une suspension de 0,85 g d'aluminohydrure de lithium dans 40 ml de tétrahydrofurane. Après 18 heures de reflux, le mélange est hydrolysé par successivement 0,6 ml d'eau, 0,5 ml de soude à 20 %, puis par 2,2 ml d'eau. Après filtration des sels minéraux, le filtrat est évaporé à l'évaporateur rotatif pour obtenir 2,5 g du produit titre. Le sel est obtenu par addition de 119 ml d'une solution d'acide (dl) dibenzoyltartrique à 2 % dans l'éthanol. On obtient, après évaporation du solvant sous vide et cristallisation dans l'eau, 3,8 g de dibenzoyltartrate (dl) de (7RS) 7-{N-[2-(3,4-diméthoxyphényl)éthyl]-N-propyl-amino}-5,6,7,8-tétrahydronaphto-[2,3-b] 2,3-dihydrofurane, PF (K) : 100-105 °C ; Rendement: 46 %.

### RMN (DMSO d6)

### Spectre ¹H :

7,95 ppm (d,4H) 7,65 ppm (t,2H) 7,5 ppm (t,4H) 6,85 ppm (m,2H); 6,80 ppm (d,1H) ; 6,75 ppm (d,1H) 6,45 ppm (s,1H) 5,7 ppm (s,2H) ; 4,45 ppm (t,2H) 3,7 ppm (2s,6H) ; 3,3 ppm (m,1H) ; 3,10 ppm (t,2H) ; 3,15 à 2,60 ppm (m,10H) ; 2,05 ppm (m,1H) ; 1,75 à 1,45 ppm (m,3H) ; 0,8 ppm (t,3H) ; échangeables non mis clairement en évidence.

### Exemple 2

### (7RS) 7-{N-[2-(4-fluorophényl)éthyl]-N-propyl-amino}-5,6,7,8-tétrahydronaphto [2,3-b] 2,3-dihydrofurane et son dibenzoyltartrate (dl)

Ce composé est obtenu comme décrit dans l'exemple 1 en remplaçant au stade A de l'exemple 1 l'acide 3,4-diméthoxyphényl acétique par l'acide 4-fluorophényl acétique. Le dibenzoyltartrate (dl) obtenu fond (K) à 115-120 °C ; Rendement : 43 %.

### RMN (DMSO d6)

### Spectre ¹H

8,00 ppm (d,4H) ; 7,65 ppm (t,2H) ; 7,5 ppm (m,4H) ; 7,25 ppm (m,2H) ; 7,1 ppm (m,2H) ; 6,9 ppm (s,1H) ; 6,4 ppm (s,1H) ; 5,75 ppm (s,2H) ; 4,45 ppm (t,2H) ; 3,45 ppm (m,1H) ; 3,20 à 2,80 ppm (m,10H) ; 2,70 ppm (m,2H) ; 2,15 à 1,65 ppm (2m,4H) ; 0,8 ppm (t,3H) ; échangeables non mis en évidence.

### Exemple 3

### (7RS) 7-{N-[2-(4-hydroxyphényl)éthyl]-N-propyl-amino}-5,6,7,8-tétrahydronaphto [2,3-b] 2,3-dihydrofurane et son dibenzoyltartrate (dl)

### Stade A

On utilise la méthode de l'exemple 1 dans laquelle l'acide 3,4-diméthoxyphényl acétique a été remplacé par de l'acide 4-benzyloxyphényl acétique.

### Stade B

On utilise la méthode de l'exemple 1 stade B

### Stade C : Composé titre

Une solution de 9 g du produit obtenu au stade précédent dans 200 ml d'éthanol est hydrogénée pendant 6 heures à température ambiante sous 200 g d'hydrogène en présence de 0,8 g de palladium sur charbon à 5 %. Après avoir absorbé la quantité requise d'hydrogène, le milieu réactionnel est filtré et concentré à l'évaporateur rotatif et traité par addition de 175 ml d'une solution d'acide (dl) dibenzoyltartrique à 2 % dans l'éthanol. Après évaporation du solvant sous vide, et cristallisation du résidu dans l'eau, on obtient 5,9 g du dibenzoyltartrate (dl) attendu qui fond (K) à 130-133 °C Rendement : 50 %.

### RMN (DMSO d6)

### Spectre ¹H

8,00 ppm (d,4H) 7,65 ppm (t,2H) ; 7,5 ppm (m,4H) ; 7,0 ppm (d,2H) ; 6,85 ppm (s,1H) ; 6,65 ppm (d,2H) ;6,45 ppm (s,1H) ; 5,75 ppm (s,2H) ; 4,45 ppm (t,2H) ; 3,45 ppm (m,1H) ; 3,2 à 2,6 ppm (m,12H) ; 2,15 ppm (m,1H) ; 1,80 à 1,50 ppm (m,3H) ; 0,8 ppm (t,3H) ; échangeables non mis évidence.

### Exemple 4

### (7RS) 7-{N-[2-(3-hydroxyphényl)éthyl]-N-propyl-amino}-5,6,7,8-tétrahydronaphto [2,3-b] 2,3-dihydrofurane et son dibenzoyltartrate (dl).

On utilise les mêmes méthodes que pour l'exemple 3 mais en remplaçant au stade A l'acide 4-benzyloxyphényl acétique par l'acide 3-benzyloxyphényl acétique. Le dibenzoyltartrate (dl) obtenu fond (K) à 128-132 °C Rendement : 46 %.

### RMN (DMSO d6)

### Spectre ¹H

8,0 ppm (d,4H) ; 7,65 ppm (t,2H) ; 7,5 ppm (m,4H) ; 7,05 ppm (t,1H) ; 6,95 ppm (s,1H) ; 6,65 ppm (m,3H) ; 6,45 ppm (s,1H) ; 5,75 ppm (s,2H) ; 4,45 ppm (t,2H) ; 3,5 ppm (m,1H) ; 3,30 à 2,50 ppm (m,12H) ; 2,15 ppm (m,1H) ; 1,80 à 1,50 ppm (m,3H) ; 0,85 ppm (t,3H) ; échangeables non mis en évidence.

### Exemple 5

### (7RS) 7-{N-[2-(3,4-dihydroxyphényl)éthyl]-N-propyl-amino}-5,6,7,8-tétrahydronaphto [2,3-b] 2,3-dihydrofurane, et son dibenzoyltartrate (dl).

On opére comme pour l'exemple 3 mais en utilisant au stade A l'acide 3,4-dibenzyloxyphényl acétique à la place de l'acide 4-benzyloxyphényl acétique. Le dibenzoyltartrate (dl) obtenu fond (K) à 124-128 °C Rendement : 34 %.

### RMN (DMSO D6)

### Spectre ¹H

9,50 à 8,50 ppm (m,2H échangeables par D₂O) ; 8,00 ppm (d,4H); 7,65 ppm (t,2H) ; 7,50 ppm (m,4H) ; 6,90 ppm (s,1H) ; 6,65 ppm (m,2H) ; 6,5 ppm (m,2H) ; 5,75 ppm (s,2H) ; 4,45 ppm (t,2H) ; 3,45 ppm (m,1H) ; 3,20 à 2,60 ppm (m,12H) ; 2,15 ppm (m,1H) ; 1,8 à 1,5 ppm (m,3H) ; 0,85 ppm (t,3H).

### Exemple 6

### (7RS) 7-{N-[2-((3H)-indol-2-one 5-yl] éthyl)-N-propyl-amino}-5,6,7,8-tétrahydronaphto [2,3-b] 2,3-dihydrofurane, et son chlorhydrate

Un mélange de 6 g de 5-(2-chloroéthyl) *(3H)*-indol-2-one et de 7,2 g de 7-N-propylamino 5,6,7,8-tétrahydronaphto [2,3-b] 2,3-dihydrofurane dans une solution de 7,5 g de carbonate neutre de sodium dans 100 ml d'eau est porté au reflux pendant 5 heures. Après une nouvelle addition de 6 g du dérivé chloré et un nouveau reflux de 36 heures, le milieu réactionnel est refroidi, extrait à l'acétate d'éthyle, décanté et évaporé à siccité. On obtient 2,2 g du produit attendu purifié par flash-chromatographie sur silice en utilisant l'acétate d'éthyle comme éluant et salifié dans 22 ml d'acétate d'éthyle par addition de 2,8 ml d'une solution d'éther chlorhydrique 2,2 N. Le chlorhydrate attendu fond (K) à 178-182 °C ; Rendement : 19 %.

### RMN (DMSO d6)

### Spectre ¹H

10,55 à 10,35 ppm (m,2H échangeables par D₂O) ; 7,20 ppm (d,1H) 7,10 ppm (d,1H); 6,95 ppm (s,1H) ; 6,75 ppm (d,1H) ; 6,55 ppm (s,1H) ; 4,45 ppm (t,2H) ; 3,65 ppm (m,1H) ; 3,5 ppm (s,2H) ; 3,40 à 2,90 ppm (m,10H) ; 2,8 ppm (m,2H) ; 2,30 ppm (m,1H) ; 1,80 ppm (m,3H) ; 0,95 ppm (t,3H).

### Exemple 7

### (7RS) 7-{N-[3-((3H)-indol-2-one-5-yl)propyl]-N-propyl-amino}-5,6,7,8-tétrahydronaphto [2,3-b] 2,3-dihydrofurane.

Ce produit a été préparé en opérant comme dans l'exemple 6 mais en utilisant le 5-(3-chloropropyl) *(3H)*-indol-2-one à la place du 5-(2-chloroéthyl) *(3H)*-indol-2-one. Le produit titre fond (K) à 126-128 °C ; Rendement : 27 %.

### RMN (CDCl₃)

### Spectre ¹H

8,1 ppm (m,1H); 7,05 ppm (m,2H); 6,9 ppm (1s,1H); 6,8 ppm (d,1H); 6,5 ppm (s,1H); 4,5 ppm (t,2H) ; 3,5 ppm (s,2H) ; 3,0 ppm (m,1H) ; 2,9 à 2,5 ppm (m,10H) ; 2,1 à 1,4 ppm (m,6H) ; 0,95 ppm (t,3H).

### Exemple 8

### (7RS) 7-{N-[2-(3H)indol-2-one-4-yl)éthyl]-N-propyl-amino}-5,6,7,8-tétrahydronaphto [2,3-b] 2,3-dihydrofurane, et son chlorhydrate.

Ce produit a été préparé en opérant comme dans l'exemple 6, en utilisant les matières premières appropriées. Le chlorhydrate du produit titre fond (MK) à 135-150 °C

### Exemple 12

### (7RS) 7-[N-(2,2-dicyclopropyl-éthyl)-N-propyl-amino]-5,6,7,8-tétrahydronaphto [2,3-b] 2,3-dihydrofurane, et son dibenzoyltartrate (dl)

### Méthode générale A : (N-alkylation par un dérivé halogéné)

A une solution de 1,3 g (5,6 mmole) de 7-(N-propylamino) 5,6,7,8-tétrahydronaphto [2,3-b] 2,3-dihydrofurane dans 20 ml d'acétonitrile, on ajoute 1,8 g (2,4 équivalents) de K₂CO₃ et 1,2 équivalent de 1-iodo-2,2-dicyclopropyl-éthane à 25 °C. On agite à température ambiante 2 heures. Puis la solution est filtrée et le filtrat évaporé à sec. Le résidu est repris par CH₂Cl₂, lavé à l'eau, séché sur MgSO₄, filtré et évaporé à sec. Le produit obtenu est purifié par flash-chromatographie sur silice (éluant : CH₂Cl₂-CH₃OH-NH₄OH: 95-5-0,5). On obtient la base sous forme d'huile.

A 2,5 mmole de base en solution dans 40 ml d'éthanol sont ajoutés 47,2 ml (1 équivalent) d'une solution à 2 % dans l'éthanol d'acide dibenzoyltartrique (dl). On agite 15 minutes puis on évapore à sec. Le résidu obtenu est cristallisé dans l'eau. Les cristaux sont alors filtrés puis séchés. Le dibenzoyltartrate obtenu fond (MK) à 90-95 °C, Rendement : 40 %.

### RMN (DMSO d6)

### Spectre ¹H

7,95 ppm (d,4H) ; 7,65 ppm (t,2H) ; 7,5 ppm (m,4H) ; 6,9 ppm (s,1H) ; 6,45 ppm (s,1H) ; 5,7 ppm (s,2H) ; 4,45ppm (t,2H) ; 3,5 ppm (m,1H) ; 3,3-2,6 ppm (m,10H) ; 2,15 ppm (m,1H) ; 1,9-1,5 ppm (m,5H) ; 0,85 ppm (t,3H) ; 0,65 ppm (m,3H) ; 0,35 à 0,1 ppm (2m,8H).

### Exemple 13

### (7RS) 7-{N-[3-(4-acétamidophényl)propyl]-N-propyl-amino}-5,6,7,8-tétrahydronaphto [2,3-b] 2,3-dihydrofurane et son dibenzoyltartrate (dl).

Ce composé a été obtenu en utilisant la méthode générale A de l'exemple 12 mais en remplaçant le 1-iodo-2,2-dicyclopropyl-éthane par le 3-(4-acétamidophényl)-1-iodo propane. Le dibenzoyltartrate obtenu fond (K) à 130-134 °C ; Rendement : 61 %.

### Exemple 14

### (7RS) 7-[N-(3,3-dicyclopropyl-propyl)-N-propyl-amino]-5,6,7,8-tétrahydronapBhto [2,3-b] 2,3-dihydrofurane et son dibenzoyltartrate (dl)

### Méthode générale B

### Stade B1 : formation de l'amide

### (7RS) 7-[N-(3,3-dicyclopropyl-propionyl)-N-propyl-amino]-5,6,7,8-tétrahydronaphto [2,3-b] 2,3-dihydrofurane

A une solution de 1,6 g (7,0 mmole) de 7-(N-propyl-amino)-5,6,7,8-tétrahydronaphto [2,3-b] 2,3-dihydrofurane dans 50 ml de dichlorométhane et en présence de 2,4 ml (2 équivalents) de N,N-diisopropyl-éthyl-amine, on ajoute à 0°C une solution de 1,7 g (10,3 mmole) de chlorure de l'acide 3,3-dicyclopropyl propionique. On agite la nuit à 25 °C. Puis on dilue à l'eau et extrait la phase organique que l'on lave par HCl (1N) puis H₂O. On sèche sur MgSO₄, on filtre et évapore à sec. On obtient 4 g d'un produit qui est purifié par flash-chromatographie sur silice (éluant : CH₂Cl₂ 100 %) pour donner 2,5 g d'amide sous forme d'huile ; Rendement: 100%.

### Stade B2: réduction par LiAlH₄

### (7RS) 7-[N-(3,3-dicyclopropyl-propyl)-N-propyl-amino]-5,6,7,8-tétrahydronaphto [2,3-b] 2,3-dihydrofurane et son dibenzoyltartrate (dl)

A une suspension de 302 mg (8,0 mmole) de LiAlH₄ dans 40 ml de tétrahydrofurane anhydre, on ajoute à 0 °C une solution de 2,0 g (5,4 mmole) d'amide du stade B1 dans 50 ml de tétrahydrofurane anhydre. On laisse remonter la température à 25 °C et on agite la nuit à température ambiante. On hydrolyse à l'eau. On filtre les sels et on évapore à sec le filtrat. On obtient 1,9 g d'un produit qui est purifié par flash-chromatographie sur silice (éluant ; CH₂Cl₂-CH₃OH : 95-5) pour donner 1,6 g de base sous forme d'huile.

A 800 mg de base (2,2 mmole) en solution dans 30 ml d'éthanol sont ajoutés 42,5 ml (1 équivalent) d'une solution à 2 % dans C₂H₅OH d'acide dibenzoyltartrique (dl). On agite 15 minutes puis on évapore à sec. Le résidu obtenu est cristallisé dans l'eau. Les cristaux sont alors filtrés puis séchés. On obtient 1,5 g du dibenzoyltartrate (dl) attendu PF (MK) : 120-148 °C ; Rendement : 93 %.

### RMN (DMSO d6)

### Spectre ¹H :

7,95 ppm (d,4H) ; 7,65 ppm (t,2H) ; 7,5 ppm (t,4H) ; 6,9 ppm (s,1H) ; 6,45 ppm (s,1H) ; 5,7 ppm (s,2H) ; 4,45 ppm (t,2H) ; 3,5 ppm (m,1H) ; 3,3 à 2,6 ppm (m,10H) ; 2,15 ppm (m,1H) ; 1,9 à 1,5 ppm (m,5H) ; 0,85 ppm (t,3H) ; 0,65 ppm (m,3H) ; 0,35-0,1 ppm (2m,8H).

### Exemple 15

### (7RS) 7-[N-(2-cyclopropyl-éthyl)-N-propyl-amino]-5,6,7,8-tétrahydronaphto [2,3-b] 2,3-dihydrofurane, et son iodhydrate.

Ce composé est obtenu en utilisant la Méthode générale B de l'exemple 14.

### Stade B1

### (7RS) 7-[N-(2-cyclopropyl-acétyl)-N-propyl-amino]-5,6,7,8-tétrahydronaphto [2,3-b] 2,3-dihydrofurane

Ce composé est obtenu en utilisant la Méthode générale B - Stade B1 de l'exemple 14 mais en remplaçant le chlorure de l'acide 3,3-dicyclopropyl propionique par le chlorure de l'acide 2-cyclopropyl acétique ; Rendement : 55 %.

### Stade B2

### (7RS) 7-[N-(2-cyclopropyl-éthyl)-N-propyl-amino]-5,6,7,8-tétrahydronaphto [2,3-b] 2,3-dihydrofurane et son iodhydrate

Ce composé est obtenu en utilisant la Méthode générale B - Stade B2 de l'exemple 14 mais lors de la formation du sel, la base est solubilisée dans le chloroforme et la solution d'acide dibenzoyltartrique (dl) à 2 % dans C₂H₅OH est remplacée par une solution d'acide iodhydrique dans le chloroforme (0,15N). Le produit est cristallisé dans l'acétate d'éthyle. L'iodhydrate obtenu fond (MK) à 144-148 °C Rendement : 40 %.

### RMN (DMSO d6)

### Spectre ¹H

6,95 ppm (s;1H) ; 6,5 ppm (s,1H) ; 4,4 ppm (t,2H) ; 3,65 ppm (m,1H) ; 3,4 à 2,6 ppm (m, 10H) ; 2,2 à 1,85 ppm (2m,2H) ; 1,8 à 1,5 ppm (m,4H) ; 0,95 ppm (t,3H) ; 0,75 ppm (m,1H) ; 0,45 à 0,15 ppm (2m,4H).

### Exemple 16

### (7RS) 7-[N-(2-cyclopentyl-éthyl)-N-propyl-amino]-5,6,7,8-tétrahydronaphto [2,3-b] 2,3-dihydrofurane et son dibenzoyltartrate (dl)

Ce composé est obtenu en utilisant la Méthode générale B - Stade B1 et Stade B2 de l'exemple 14.

### Stade B1

### (7RS) 7-[N-(2-cyclopentyl-acétyl)-N-propyl-amino]-5,6,7,8-tétrahydronaphto [2,3-b] 2,3-dihydrofurane

Ce composé est obtenu en utilisant la Méthode générale B - Stade B1 de l'exemple 14 mais en remplaçant le chlorure de l'acide 3,3-dicyclopropyl propionique par le chlorure de l'acide 2-cyclopentyl acétique ; Rendement : 100 %.

### Stade B2

### (7RS) 7-[N-(2-cyclopentyl-éthyl)-N-propyl-amino]-,5,6,7,8-tétrahydronaphto [2,3-b] 2,3-dihydrofurane et son dibenzoyltartrate (dl)

Ce composé est obtenu en utilisant la Méthode générale B - Stade B2 de l'exemple 14. Le dibenzoyltartrate obtenu fond (MK) à 95-100 °C Rendement : 59 %.

### RMN (DMSO d6)

### Spectre ¹H

7,95 ppm (d,4H) 7,65 ppm (m,2H) ; 7,5 ppm (m,4H) ; 6,9 ppm (s,1H) ; 6,45 ppm (s,1H) ; 5,7 ppm (s,2H) ; 4,45 ppm (t,2H) ; 3,5 ppm (m,1H) ; 3,1 ppm (t,2H) ; 3,1 à 2,6 ppm (2m,8H) ; 2,15 ppm (m,1H) ; 1,9 à 1,4 ppm (m,12H) ; 1,1 ppm (m,2H) ; 0,85 ppm (t,3H).

### Exemple 17

### (7RS) 7-{N-[4-(p-bromo-benzamide)-butyl]-N-propyl-amino}-5,6,7,8-tétrahydronaphto [2,3-b] 2,3-dihydrofurane, et son chlorhydrate

On dissout dans 80 ml de 1,2-dichloroéthane 3,3 g (14,2 mmole) de 7-(N-propyl-amino)-5,6,7,8-tétrahydronaphto [2,3-b] 2,3-dihydrofurane. On ajoute 4,2 g (15,7 mmole) de (p-bromo-benzamide)-butyraldéhyde, puis 0,8 ml (14,2 mmole) d'acide acétique. On refroidit à +10 °C et on ajoute 4,5 g (21,3 mmole) de triacétoxy borohydrure de sodium. On agite une nuit à température ambiante. On ajoute 100 ml d'eau, extrait la phase organique, sèche sur MgSO₄, filtre et évapore à sec. On obtient 7 g d'un produit qui est purifié par flash-chromatographie sur silice (éluant CH₂Cl₂-CH₃OH-NH₄OH : 95-5-0,5) pour donner 2 g de base sous forme d'huile.
Ces 2g (4,1 mmole) de base sont solubilisés dans 100 ml d'éther et 20 ml d'acétate d'éthyle, puis on ajoute 2 ml (5,0 mmole) d'une solution d'éther chlorhydrique 2,5 N. Le solide qui précipite est filtré et séché. On obtient 1,4 g du chlorhydrate attendu qui fond (MK) à 100-115 °C ; Rendement : 20 %.

### RMN (DMSO d6)

### Spectre ¹H

10,1 ppm (m,NH+) ; 8,7 ppm (t,NH) ; 7,85 ppm (d,2H) ; 7,7 ppm (d,2H) ; 7,0 ppm (s,1H) ; 6,5 ppm (s,1H) ; 4,5 ppm (t,2H) ; 3,6 ppm (m,1H) ; 3,5 à 2,7 ppm (m,2H) ; 2,3 ppm (m,1H) ; 2,0 à 1,5 ppm (m,7H) ; 1,0 ppm (t,3H).

### Exemple 18

### (7RS) 7-{N-[4-(cyclohexylamide)butyl]-N-propyl-amino}-5,6,7,8-tétrahydronaphto[2,3-b] 2,3-dihydrofurane et son dibenzoyltartrate (dl).

Ce composé est préparé selon la méthode décrite dans l'exemple 17 mais en remplaçant la 4-(p-bromobenzamide)butyraldéhyde par la 4-(cyclohexylamide)butyraldéhyde. Le dibenzoyltartrate attendu fond (MK) à 108-111 °C ; Rendement : 34 %.

### Exemple 19

### (7RS) 7-{N-[4-(p-fluorobenzamide)butyl]-N-propyl-amino}-5,6,7,8-tétrahydronaphto [2,3-b] 2,3-dihydrofurane et son dibenzoyltartrate (dl)

Ce composé est préparé selon la méhode décrite dans l'exemple 17 mais en remplaçant la 4-(p-bromobenzamide)butyraldéhyde par la 4-(p-fluorobenzamide)butyraldéhyde. Le dibenzoyltartrate attendu fond (MK) à 108-114 °C ; Rendement : 19 %.

### Exemple 20

### (7RS) 7-{N-[4-(m-trifluorométhylbenzamide)butyl]-N-propyl-amino}-5,6,7,8-tétrahydro naphto [2,3-b] 2,3-dihydrofurane et son dibenzoyltartrate (dl)

Ce composé est préparé selon la méthode décrite dans l'exemple 17 mais en remplaçant la 4-(p-bromobenzamide)butyraldéhyde par la 4-(m-trifluorométhyl)butyraldéhyde. Le dibenzoyltartrate fond (MK) à 100-105 °C Rendement : 38 %.

### Exemples 21-25

En opérant selon la méthode de l'exemple 17, à partir des matières premières appropriées, ont également été préparés les composés objet des exemples suivants :
21) (7RS) 7-{N-[4-(p-cyanobenzamide)butyl]-N-propyl-amino}-5,6,7,8-tétrahydronaphto [2,3-b] 2,3-dihydrofurane, dont le dibenzoyltartrate fond (MK) à 112-117 °C.
22) (7RS) 7-{N-[3-(p-bromobenzamide)propyl]-N-propyl-amino}-5,6,7,8-tétrahydronaphto [2,3-b] 2,3-dihydrofurane, dont le dibenzoyltartrate fond (MK) à 116-124 °C.
23) (7RS) 7-{N-[4-(o-bromobenzamide)butyl]-N-propyl-amino}-5,6,7,8-tétrahydronaphto [2,3-b] 2,3-dihydrofurane, dont le dibenzoyltartrate fond (MK) à 112-115 °C.
24) (7RS) 7-{N-[4-(p-trifluorométhylbenzamide)butyl]-N-propyl-amino}-5,6,7,8-tétrahydro naphto [2,3-b] 2,3-dihydrofurane, dont le dibenzoyltartrate fond (MK) à 102-108 °C.
25) (7RS) 7-{N-[3-(p-cyanobenzamide)propyl]-N-propyl-amino}-5,6,7,8-tétrahydronaphto [2,3-b] 2,3-dihydrofurane, dont le dibenzoyltartrate fond (MK) à 129-136 °C.

### Exemple 26

### (7RS) 7-[N-(2-cyclobutyl-éthyl)-N-propyl-amino]-5,6,7,8-tétrahydronaphto [2,3-b] 2,3-dihydrofurane, et son dibenzoyltartrate (dl)

### Méthode générale C

### Stade C1

### (7RS) 7-[N-(2-cyclobutyl-acétyl)-N-propyl-amino]-5,6,7,8-tétrahydronaphto [2,3-b] 2,3-dihydrofurane

Ce composé est obtenu en utilisant la Méthode générale B - Stade B1 de l'exemple 14 mais en remplaçant le chlorure de l'acide 3,3-dicyclopropyl propionique par le chlorure de l'acide 2-cyclobutyl acétique.Le produit attendu fond (K) à 84-86 °C, Rendement : 41 %.

### Stade C2

### (7RS) 7-[N-(2-cyclobutyl-éthyl)-N-propyl-amino]-5,6,7,8-tétrahydronaphto [2,3-b] 2,3-dihydrofurane et son dibenzoyltartrate (dl)

A 2,3 g (7,0 mmole) d'amide du stade C1 solubilisés dans 200ml de tétrahydrofurane anhydre, on ajoute 2,6 ml (4 équivalents) de BH₃.(CH₃)₂S (10 M). Le mélange est chauffé 2 heures à reflux. On refroidit à 0 °C, et on ajoute avec précaution 20 ml de CH₃OH-HCl (2,2 N). On agite une nuit à température ambiante. Puis le mélange réactionnel est évaporé à sec. Le résidu est repris dans 100 ml d'eau. On porte le pH à 8 en ajoutant NaOH (1N) et extrait à l'acétate d'éthyle. La phase organique est séchée sur MgSO₄, filtrée et évaporée à sec. On obtient 2 g d'un produit qui est purifié par flash-chromatographie sur silice (éluant : CH₂Cl₂-CH₃OH : 95-5) pour donner 1,2 g de base sous forme d'huile.
A 500 mg de base (1,6 mmole) en solution dans 30 ml d'éthanol sont ajoutés 29,9 ml (1 équivalent) d'une solution à 2 % dans C₂H₅OH d'acide dibenzoyltartrique (dl). On agite 15 minutes puis évapore à sec. Le résidu obtenu est cristallisé dans l'eau. Les cristaux sont alors filtrés puis séchés. On obtient 950 mg du dibenzoyltartrate attendu, PF (MK) : 105-110 °C, Rendement : 45 %.

### RMN (DMSO 6d)

### Spectre ¹H

7,95 ppm (d,4H) ; 7,65 ppm (t,2H) ; 7,5 ppm (t,4H) ; 6,95 ppm (s,1H) ; 6,45 ppm (s,1H) ; 5,7 ppm (s,2H) ; 4,45 ppm (t,2H) ; 3,45 ppm (m,1H) ; 3,1 ppm (t,2H) ; 2,85 ppm (m,4H) ; 2,85 à 2,7 ppm (m,4H) ; 2,25 à 2,17 ppm (3m,13H) ; 0,85 ppm (t,3H).

### Exemple 27

### (7RS) 7-[N-(2-cyclohexyl-éthyl)-N-propyl-amino]- 5,6,7,8-tétrahydronaphto [2,3-b] 2,3-dihydrofurane, et son dibenzoyltartrate (dl)

### Stade C1

### (7RS) 7-[N-(2-cyclohexyl-acétyl)-N-propyl-amino]-5,6,7,8-tétrahydronaphto [2,3-b] 2,3-dihydrofurane

Ce composé est obtenu en utilisant la Méthode générale B - Stade B1 de l'exemple 14 mais en remplaçant le chlorure de l'acide 3,3-dicyclopropyl propionique par le chlorure de l'acide 2-cyclohexyl acétique, Rendement : 95 %.

### Stade C2

### (7RS) 7-[N-(2-cyclohexyl-éthyl)-N-propyl-arnino]-5,6,7,8-tétrahydronaphto [2,3-b] 2,3-dihydrofurane

Ce composé est obtenu en utilisant la Méthode générale C - Stade C2 de l'exemple 26 PF(MK) : 95-102 °C ; Rendement : 20 %.

### RMN (DMSO d6)

### Spectre ¹H

8,0 ppm (d,4H) ; 7,65 ppm (t,2H) ; 7,5 ppm (m,4H) ; 6,9 ppm (s,1H) ; 6,45 ppm (s,1H) ; 5,65 ppm (s,2H) ; 4,45 ppm (t,2H) ; 3,4 ppm (m,1H) ; 3,1 ppm (t,2H) ; 3,1 à 2,6 ppm (m,8H) ; 2,1 ppm (m,1H) ; 1,7 à 1,0 ppm (m,5H) ; 1,4 à 0,7 ppm (m,11H) ; 0,8 ppm (t,3H).

### Exemple 28

### (7RS) 7-[N-(cis-2-méthylcyclopropyl-méthyl)-N-propyl-amino]-5,6,7,8-tétrahydronaphto [2,3-b] 2,3-dihydrofurane et son dibenzoyltartrate (dl)

Ce composé est obtenu en utilisant la méthode générale C, décrite dans l'exemple 26, mais en utilisant au stade C1 le chlorure de l'acide cis-2-méthylcyclopropane carboxylique à la place du chlorure de l'acide 2-cyclobutyl acétique. Le dibenzoyltartrate attendu fond (MK) à 95-100 °C; Rendement : 38 %.

### Exemple 29

### (7RS) 7-{N-[2-(biphényl-4-yl)éthyl]-N-propyl-amino}-5,6,7,8-tétrahydronaphto[2,3-b] 2,3-dihydrofurane et son dibenzoyltartrate (dl)

Ce composé est obtenu en utilisant la méthode générale C décrite dans l'exemple 26, mais en utilisant au stade C1 le chlorure de l'acide 2-(biphényl-4-yl)acétique à la place du chlorure de l'acide 2-cyclobutyl acétique. Le dibenzoyltartrate attendu fond (MK) à 100-105 °C ; Rendement : 35 %.

### Exemple 30

### Etude Pharmacologique

### ETUDES DE LA LIAISON DES COMPOSES DE L'INVENTION AUX RECEPTEURS HUMAINS D₂ ET D₃ EXPRIMES DANS LES CELLULES CHO

### 1/ Matériel et méthode

### • Culture cellulaire

Les cellules CHO (Chinese Hamster Ovary) ont été transfectées de façon stable par le gène codant pour le récepteur humain de la dopamine D₂ ou D₃ selon des méthodes connues de la littérature. Les cellules natives sont déficientes en enzyme DHFR (DiHydroFolate Reductase). Ces cellules sont cultivées dans une étuve à 37 °C, en atmosphère humide 5 % CO₂, 95 % air. Les transfections ont été réalisées en utilisant la Lipofectine (Gibco). Les cellules CHO cotransfectées avec le récepteur D₂ humain et le gène de résistance à la phléomycine ont été sélectionnées pour leur résistance à la présence de cet antibiotique dans le milieu de culture. Les cellules transfectées avec le récepteur D₃ humain ont été sélectionnées dans un milieu dépourvu d'hypoxanthine/thymidine, en présence de methotrexate. La composition des milieux de culture utilisés sont pour les CHO-D₂ : DMEM (Dulbecco's Modified Eagle Medium) supplémentés à 10 % en sérum de veau foetal et en hypoxanthine/thimidine et pour les CHO-D₃ : DMEM supplémentés à 10 % en sérum de veau foetal dialysé. Les cellules sont récoltées à confluence et les membranes sont alors préparées.

### • Préparation membranaire

Après quelques minutes en présence de trypsine 0,2 %, les cellules sont récupérées et centrifugées à 2 000 g pendant 5 minutes. Le culot cellulaire, resuspendu dans du tampon Tris-HCl 10 mM, pH 7,5 contenant 5mM de MgSO₄, est alors passé au Polytron® .

L'homogénat est ensuite centrifugé à 50 000 g pendant 15 minutes, et le culot est resuspendu par sonication douce dans le tampon d'incubation de composition : 50 mM de tris-HCl, pH 7.5 contenant 120 mM de NaCl, 5mM de KCI, 2mM de CaCl₂ et 5 mM de MgCl₂. Les membranes sont ensuite aliquotées et conservées à -80 °C jusqu'au jour de l'expérience.

### • Expériences de liaison

Les incubations sont effectuées dans des tubes en polypropylène pour un volume final de 400 µl contenant :
100 µl de [¹²⁵I]-iodosulpride (Amersham)
   à 0,1 et 0,2 nM pour les récepteurs D₂ et D₃ respectivement.
100 µl de tampon (tubes totaux)
   ou
100 µl de raclopride 10 µM (liaison non spécifique)
   ou
100 µl de composé.
200 µl de préparation membranaire contenant les récepteurs D₂ ou D₃, dans du tampon additionné de 0,2 % de BSA (Bovine Serum Albumine).

Les gammes de concentration de chaque composé comportent au moins 7 points déterminés en triple, chaque expérience est répétée au moins 2 fois.

L'incubation qui dure 30 minutes à 30 °C est stoppée par une filtration rapide sur appareil Brandle, suivie de 3 rinçages consécutifs avec du tampon Tris-HCl pH 7,4 contenant 120 mM de NaCl. Les filtres récupérés sont ensuite comptés au compteur gamma.

### • Analyse des résultats

L'IC₅₀ représentant la concentration donnant 50 % d'inhibition de la liaison du radioligand est calculée par régression non linéaire (méthode Prism Graph).
La valeur de Ki est dérivée de la formule Ki = IC₅₀/(1+L/Kd) où L est la concentration de [¹²⁵I]-Iodosulpride utilisée dans l'expérience et Kd sa constante de dissociation. Les résultats sont exprimés sous la forme de pKi (pKi = -logKi).
Pour les récepteurs humains D₂ et D_{3,} les Kd sont respectivement égaux à 0,5 et 1,31 nM.

### 2/ Résultats

Les affinités pour le récepteur humain D₃, pour des produits particulièrement représentatifs de l'invention, sont rapportés dans le tableau ci-après.

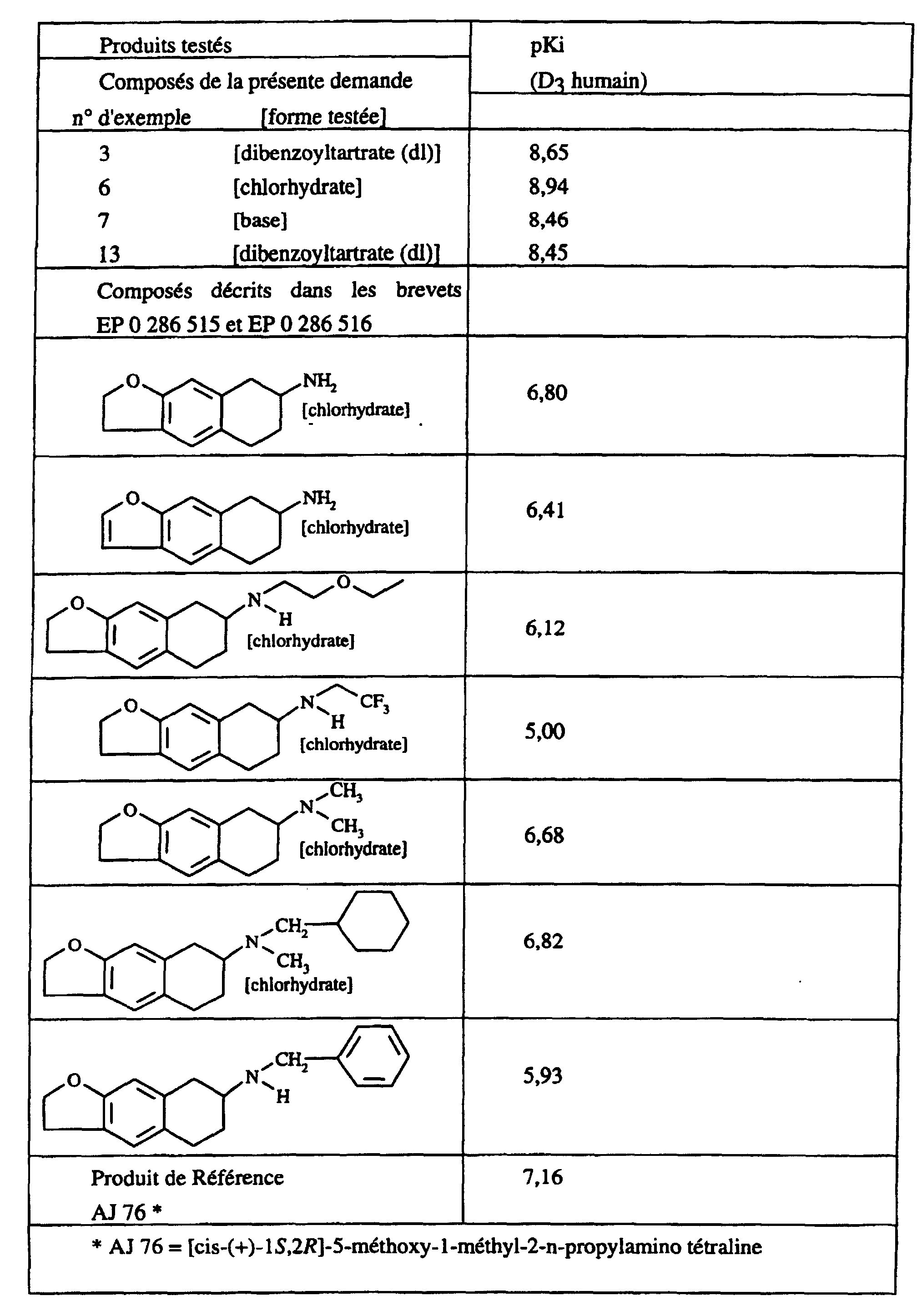

La comparaison des résultats obtenus avec les produits testés de la présente invention et ceux obtenus avec les produits décrits dans les brevets EP 0 286 515 et 0 286 516 montre que les modifications effectuées sur les substituants de la fonction amine ont apportés une augmentation d'activité très significative en ce qui concerne l'affinité pour les récepteurs D₃.

De plus, tous les produits de la présente invention montrent des affinités pour le récepteur D₂ de 10 à 100 fois inférieures à celles présentées pour le récepteur D₃, ce qui n'est pas le cas des produits décrits dans les brevets EP 0 286 515 et 0 286 516. Il en est d'ailleurs de même pour le produit de référence AJ 76 qui lui, ne présente qu'une sélectivité de 2, pour le récepteur D₃, vis à vis du récepteur D₂.

## Revendications

1. Les composés aminés de 5,6,7,8-tétrahydronaphto [2,3-b]furane et d'indano [5,6-b] furane de formule I : 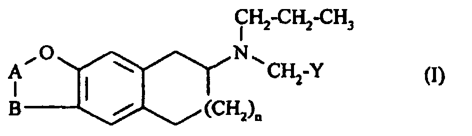 dans laquelle :
**A-B** représente CH=CH ou CH₂-CH₂ ;
**n** représente zéro ou un ; et
**Y** représente :
- un radical ω-(cycloalkyl)-alkyle, ω-(méthyl cylcoalkyl)-alkyle ou ω,ω-(di-cycloalkyl)-alkyle dans chacun desquels chaque groupe cycloalkyl renferme de 3 à 7 atomes de carbone et la partie alkyle renferme de 1 à 4 atomes de carbone en chaîne droite ou ramifiée ; ou
- un radical de formule : 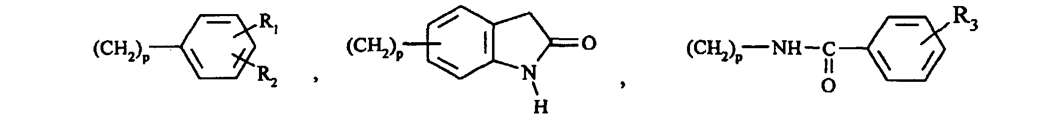 et 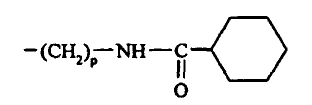 dans lesquelles :
p est un nombre entier de 1 à 4 inclus,
R₁ représente un atome d'hydrogène ou d'halogène, ou un radical hydroxy ou méthoxy,
R₂ représente un atome d'halogène, un radical hydroxy, (C₁-C₅)alkoxy ou phényle ou un groupement de formule : -NH-CO-CH₃, -NH-CO-CF₃ et -NH-SO₂-CH₃, et
R₃ représente un atome d'halogène ou un radical (C₁-C₅)alkoxy, trifluorométhyle, cyano, phényle, p-aminophényle ou p-acétylphényle ;
sous forme racémique ou d'isomères optiques,
et leurs sels d'addition avec un acide pharmaceutiquement acceptable.

2. Un composé de la revendication 1 qui est le (7RS) 7-{N-[3-(*(3H)*-indol-2-one-5-yl) propyl] N-propyl-amino}-5,6,7,8-tétrahydronaphto [2,3-b] 2,3-dihydrofurane.

3. Un composé de la revendication 1 qui est le (7RS) 7-[N-(2-cyclopentyl-éthyl)-N-propyl-amino]-5,6,7,8-tétrahydronaphto [2,3-b] 2,3-dihydrofurane et son dibenzoyltartrate (dl).

4. Un composé de la revendication 1 qui est le (7RS) 7-{N-[3-(4-acétamidophényl)propyl]-N-propyl-amino}-5,6,7,8-térahydronaphto[2,3-b] 2,3-dihydrofurane et son dibenzoyltartrate (dl).

5. Un composé de la revendication 1 qui est le (7RS) 7-{N-[4-(p-fluorobenzamide)butyl]-N-propyl-amino}-5,6,7,8-tétrahydronaphto [2,3-b] 2,3-dihydrofurane et son dibenzoyltartrate (dl).

6. Le procédé de préparation des composés de la revendication 1 **caractérisé en ce que** l'on fait réagir l'amine secondaire de formule II : 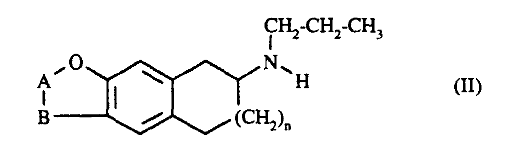 dans laquelle A-B et n ont les significations définies dans la revendication 1, avec
- soit un composé halogéné de formule III:
X-CH₂-Y (III)
dans laquelle :
Y a la signification définie dans la revendication 1 et
X représente un atome de chlore, brome ou iode,
en opérant en milieu alcalin ;
- soit avec un composé de formule IV : 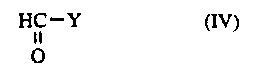 dans laquelle Y a la signification définie dans la revendication 1,
en opérant en milieu réducteur ;
- soit avec un composé acylant de formule V : 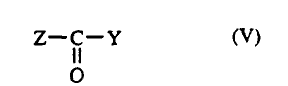 dans laquelle :
Y a la signfication définie dans la revendication 1 et
Z représente un radical hydroxy ou un atome de chlore,
et l'on réduit le composé obtenu de formule VI : 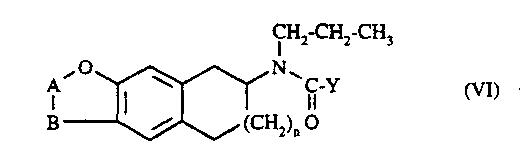
dans laquelle A-B, n et Y ont les significations précédemment définies.

7. Les compositions pharmaceutiques,utilisables dans le traitement de la maladie de Parkinson, des troubles de la mémoire, des troubles liés à l'abus de drogue, de la dépression et des états psychotiques, contenant comme principe actif au moins un composé selon l'une quelconque des revendications 1 à 5, avec un ou plusieurs excipients pharmaceutiques appropriés.

## Claims

1. 5,6,7,8-tetrahydronaphtho[2,3-b]furan and indano[5,6-b]furan amine compounds of formula I : 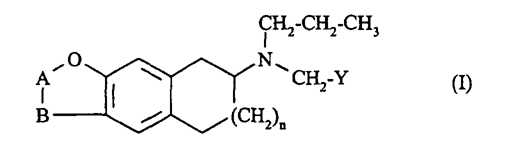 wherein :
**A-B** represents CH=CH or CH₂-CH₂;
**n** represents zero or one ; and
**Y** represents :
- a ω-(cycloalkyl)alkyl, ω-(methylcycloalkyl)alkyl or ω,ω-(dicycloalkyl)alkyl radical in each of which each cycloalkyl group contains from 3 to 7 carbon atoms and the alkyl moiety contains from 1 to 4 carbon atoms in straight or branched chain; or
- a radical of formula : 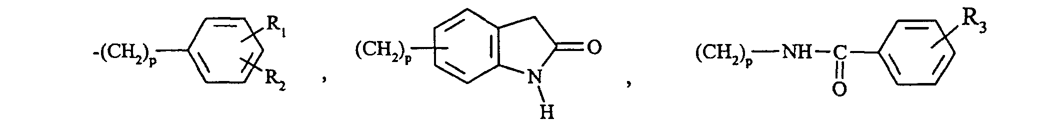 or 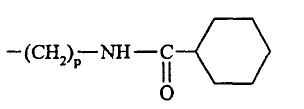 wherein :
p is an integer of from 1 to 4 inclusive,
R₁ represents a hydrogen or halogen atom, or a hydroxy or methoxy radical,
R₂ represents a halogen atom, a hydroxy, (C₁-C₅)alkoxy or phenyl radical or a group of formula : -NH-CO-CH₃, -NH-CO-CF₃ or -NH-SO₂-CH₃, and
R₃ represents a halogen atom or a (C₁-C₅)alkoxy, trifluoromethyl, cyano, phenyl, p-aminophenyl or p-acetylphenyl radical ;
in racemic form or in the form of optical isomers;
and their addition salts with a pharmaceutically acceptable acid.

2. A compound of claim 1 which is (7RS)-7-{N-[3-(*(3H)*-indol-2-on-5-yl)propyl]-N-propylamino}-5,6,7,8-tetrahydronaphtho[2,3-b]2,3-dihydrofuran.

3. A compound of claim 1 which is (7RS)-7-[N-(2-cyclopentylethyl)-N-propylamino]-5,6,7,8-tetrahydronaphtho[2,3-b]2,3-dihydrofuran and its (d1)-dibenzoyltartrate.

4. A compound of claim 1 which is (7RS)-7-{N-[3-(4-acetamidophenyl)propyl]-N-propyl-amino}-5,6,7,8-tetrahydronaphtho[2,3-b]2,3-dihydrofuran and its (dl)-dibenzoyltartrate.

5. A compound of claim 1 which is (7RS)-7-{N-[4-(p-fluorobenzamide)butyl] -N-propyl-amino}-5,6,7,8-tetrahydronaphtho[2,3-b]2,3-dihydrofuran and its (dl)-dibenzoyltartrate.

6. A process for the preparation of compounds of formula I, **characterised in that** a secondary amine of formula II : 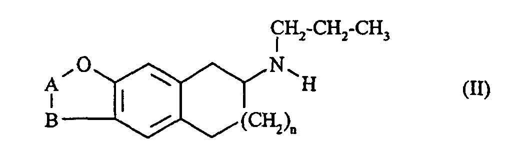 wherein A-B and n are as defined in claim 1, is reacted
- with a halogenated compound of formula III :
X-CH₂-Y (III)
wherein :
Y is as defined in claim 1 and
X represents a chlorine, bromine or iodine atom,
in an alkaline medium ;
- or with a compound of formula IV : 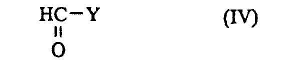
wherein Y is as defined in claim 1,
in a reductive medium ;
- or with an acylating compound of formula V : 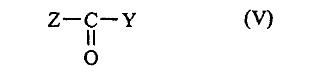
wherein :
Y is as defined in claim 1 and
Z represents a hydroxy radical or a chlorine atom,
and the resulting compound of formula VI : 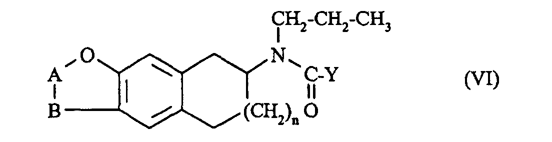 wherein A-B, n and Y are as defined above is reduced.

7. Pharmaceutical compositions that can be used in the treatment of Parkinson's disease, memory disorders, disorders associated with drug abuse, depression and psychotic states, comprising as active ingredient at least one compound according to any one of claims 1 to 5, together with one or more appropriate pharmaceutical excipients.

## Patentansprüche

1. Aminierte Verbindungen von 5,6,7,8-Tetrahydronaphtho[2,3-b]furan und Indano[5,6-b]furan der Formel I: 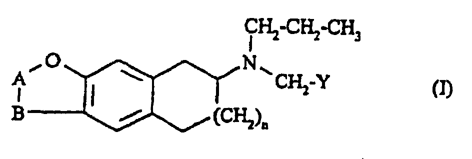 in der:
**A-B** CH=CH oder CH₂-CH₂ bedeutet;
**n** Null oder 1 bedeutet; und
**Y:**
- eine ω-(Cycloalkyl)-alkyl-, ω-(Methylcycloalkyl)-alkyl- oder ω,ω-(Di-cycloalkyl)-alkyl-Gruppe, bei der jede Cycloalkylgruppe 3 bis 7 Kohlenstoffatome aufweist und der Alkylrest 1 bis 4 Kohlenstoffatome in gerader oder verzweigter Kette umfaßt; oder
- eine Gruppe der Formel: 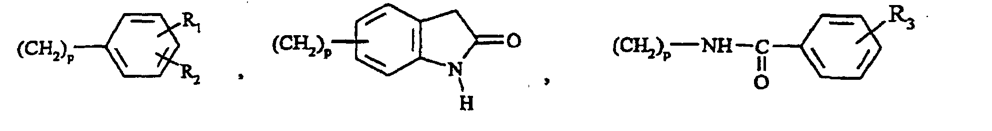 und 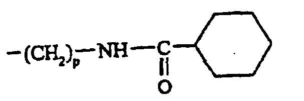
in denen:
p eine ganze Zahl mit einem Wert von 1 bis 4 einschließlich.
R₁ ein Wasserstoffatom, ein Halogenatom, eine Hydroxygruppe oder eine Methoxygruppe,
R₂ ein Wasserstoffatom, eine Hydroxygruppe, eine (C₁-C₅)-Alkoxygruppe oder eine Phenylgruppe oder eine Gruppe der Formel: -NH-CO-CH₃, -NH-CO-CF₃ und -NH-SO₂-CH₃, und
R₃ ein Halogenatom oder eine (C₁-C₅)-Alkoxygruppe, Trifluormethylgruppe, Cyanogruppe, Phenylgruppe, p-Aminophenyl- oder p-Acetylphenyl-Gruppe darstellen; bedeutet;
in racemischer Form oder in Form der optischen Isomeren,
und deren Additionssalze mit einer pharmazeutisch annehmbaren Säure.

2. Verbindung nach Anspruch 1, nämlich (7RS)-7-{N-[3-((*3H*)-indol-2-on-5-yl)-propyl]-N-propyl-amino}-5,6,7,8-tetrahydronaphtho[2,3-b]-2,3-dihydrofuran.

3. Verbindung nach Anspruch 1, nämlich (7RS)-7-[N-(2-cyclopentyl-ethyl)-N-propyl-amino]-5,6,7,8-tetrahydronaphtho[2,3-b]-2,3-dihydrofuran und dessen Dibenzoyltartrat (dl).

4. Verbindung nach Anspruch 1, nämlich (7RS)-7-{N-[3-(4-acetamidophenyl)-propyl]-N-propyl-amino}-5,6,7,8-tetrahydronaphtho[2,3-b]-2,3-dihydrofuran und dessen Dibenzoyltartrat (dl).

5. Verbindung nach Anspruch 1, nämlich (7RS)-7-{N-[4-(p-fluorbenzamid)-butyl]-N-propyl-amino}-5,6,7,8-tetrahydronaphtho[2,3-b]-2,3-dihydrofuran und dessen Dibenzoyltartrat (dl).

6. Verfahren zur Herstellung der Verbindungen nach Anspruch 1, **dadurch gekennzeichnet, daß** man ein sekundäres Amin der Formel II: 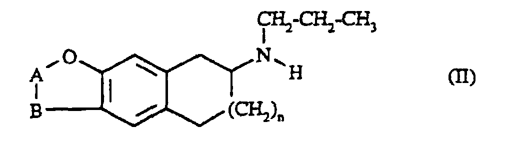 in der A-B und n die in Anspruch 1 angegebenen Bedeutungen besitzen, mit
- entweder einer Halogenverbindung der Formel III:
X-CH₂-Y (III)
in der
**Y** die in Anspruch 1 angegebenen Bedeutungen besitzt und
**X** ein Chlor-, Brom- oder Iod-Atom bedeutet,
in alkalischem Medium umsetzt;
- oder mit einer Verbindung der Formel IV: 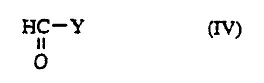 in der Y die in Anspruch 1 angegebenen Bedeutungen besitzt,
in einem reduzierenden Medium umsetzt;
- oder mit einer acylierenden Verbindung der Formel V: 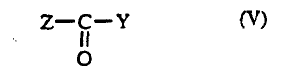 in der:
**Y** die in Anspruch 1 angegebene Bedeutung besitzt und
**Z** eine Hydroxygruppe oder ein Chloratom bedeutet,
umsetzt,
und die erhaltene Verbindung der Formel VI: 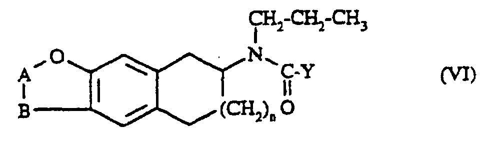 in der A-B, n und Y die oben angegebenen Bedeutungen besitzen, reduziert.

7. Pharmazeutische Zubereitungen für die Behandlung der Parkinsonschen Krankheit, von Gedächtnisstörungen, von Störungen, die mit dem Drogenmißbrauch verknüpft sind, von Depressionen und psychotischen Zuständen, enthaltend als Wirkstoff mindestens eine Verbindung nach einem der Ansprüche 1 bis 5 zusammen mit einem oder mehreren geeigneten pharmazeutischen Trägermaterialien.
